# EUROPEAN PATENT SPECIFICATION

(11) **EP 2 124 791 B1**
(45) Date of publication and mention of the grant of the patent: **13.08.2014**
(21) Application number: 07805652.0
(22) Date of filing: 20.06.2007
(51) Int. Cl.: A61B 18/02, A61M 35/00

(54) **DISPENSING DEVICE FOR PRESSURIZED CONTAINERS FOR THE APPLICATION OF CRYOGENIC COOLANT**
SPENDEVORRICHTUNG FÜR DRUCKBEHÄLTER ZUM AUFBRINGEN EINES KRYOGENEN KÜHLMITTELS
DISPOSITIF DISTRIBUTEUR POUR RECIPIENTS PRESSURISES POUR L'APPLICATION D'UN REFRIGERANT CRYOGENIQUE

(30) Priority: 31.01.2007 IT PD20070025
(43) Date of publication of application: 02.12.2009
(73) Proprietor: Sixtem Life S.r.l., 50019 Sesto Fiorentino (IT)
(72) Inventor: CITTERIO, Mauro, I-59100 Prato (PO) (IT); OTTANELLI, Luciano, I-59100 Prato (PO) (IT)
(74) Representative: Vinci, Marcello
(86) International application number: PCT/IT2007/000440
(87) International publication number: WO 2008/093372

(56) References cited:
- EP-A- 0 695 522
- EP-B- 1 065 980
- WO-A-2006/004407
- WO-A-2007/009282
- GB-A- 1 255 666
- US-A1- 2005 178 802

## Description

The present patent concerns medical devices for cryotherapy in the treatment of skin lesions and warts, and in particular it concerns a new dispensing device for pressurized containers for the application of cryogenic coolant.

Cryotherapy is a therapeutic process in which very cold fluids are used for the local treatment of skin lesions like warts or other less or more serious skin diseases.

The controlled and punctual application of coolant to the lesion causes the freezing of the intracellular water and the alteration of proteins and enzymes causing, over the therapeutic application and successive thawing time, the detachment of the epidermis from the dermis without damaging the latter. The advantages offered by cryotherapy are therefore numerous. First of all, anaesthesia of the area to be treated is not usually required and furthermore, in most cases, no scars appear and there is no need for further post-operative medications or treatments.

One of the fluids most used in cryotherapy is liquid nitrogen at the temperature of -196°C, while the use of freon, which is an halogenated gas, has been recently restricted because it is considered a pollutant.

The use of other fluids like carbon dioxide and nitrous oxide is also known, which however require more complex and expensive preparation and conservation processes.

At present, the coolant to be used in cryotherapy is kept in pressurized containers provided with spray dispensers with manual opening/closing valve.

The known containers also comprise a removable application duct or tube, at whose end there is an applicator pad suited to absorb and convey the coolant that has flowed out of the container.

Said applicator pad soaked with coolant is applied to the area to be treated, which thus freezes, causing the breakage of the cellular membranes and the successive detachment of the epidermis from the dermis.

In the known containers, said duct or tube is removably applied to the dispenser at the moment of use, which means that the user must first of all apply the tube, then position the pad and finally dispense and apply the coolant.

One of the drawbacks posed by the known devices lies in that the coolant may be spread out in an uncontrolled manner, for example during application or removal of the tube, thus causing a waste of material and above all the risk of injuries or lesions to the operator or the patient. Furthermore, it is necessary for the operator to act rapidly when dispensing the coolant, in order to prevent its temperature to increase excessively compared to the optimal values, which would reduce the effectiveness of the treatment.

The use of the known containers, which comprise removable parts like tubes and pads, often makes the operation rather complex and for this reason the operator works less rapidly.

Furthermore, he/she must wait the technical time to make sure that the pad is completely and sufficiently soaked with coolant before proceeding to the application.

EP-B-1065980 relates to a device for dispensing fluid coolant and comprising a container for the cooling agent, a valve for closing said container, means for temporary opening the valve and an administering element comprising an absorption element. The device also comprises a chamber connected to said valve and in which the cooling fluid is carried, said chamber being suitable for positioning said absorption element. US-A-2005/178802 relates to a hand-held dispenser comprising a valve and a nozzle that moves between an unactuated position and a discharge position. The dispenser also comprises a dome interacting with the nozzle and suited to rotate between a locked position and an unlocked position.

In order to overcome the drawbacks described above, a new type of dispensing device for pressurized containers has been designed and carried out, which is suitable for the application of cryogenic cooling gas in the therapeutic treatment of skin lesions.

The main object of the invention is to provide a dispensing device that can be used in total safety for the operator and the patient, since uncontrolled leakages of coolant are prevented by an activator cap or plug with which the fluid is dispensed in a closed space in order to soak the pad or applicator filter.

Another object of the present invention is to provide a device that can be used in a quick and straightforward manner, since the duct or tube of the coolant to be dispensed is built in and integral with the body of the dispenser and therefore it is not necessary to install the tube in advance, a procedure which causes inevitable time waste and possible uncontrolled fluid leakages.

Good hygienic conditions are guaranteed by the possibility to replace the pad or filter partially housed in said tube after every application.

A further object of the present invention is to speed up the application procedure, thus reducing to a minimum any decrease in the fluid temperature before application to the skin and thus increasing the effectiveness of cryotherapy to the benefit of the patient.

One of the advantages offered by the present invention, resulting from the use of pads or filters made of a highly absorbing material with high capacity to convey the coolant, lies in that it is possible to obtain the immediate cooling of the pad.

A further advantage offered by the present invention lies in that the operator can identify quickly and with certainty the moment when the pad or filter is properly soaked with coolant and therefore ready for immediate application. A further advantage of the invention lies in that it is made of materials that have suitable mechanical and chemical resistance to the substances used and are resistant to low operating temperatures.

These and other direct and complementary objects have been achieved through the construction of the new dispensing device for pressurized containers with manual valve for the application of cryogenic cooling gas in cryotherapy, comprising a dispenser body with built-in and integral tube, in which it is possible to partially insert a removable cylindrical applicator pad or filter, suited to absorb and covey the coolant, and wherein a safety activator cap or plug is suited to close the dispenser body, thus preventing accidental fluid leakages, or to open the valve causing the controlled delivery of the fluid that, conveyed through said tube, is immediately absorbed by said pad or filter.

Said activator cap comprises a casing suited to be positioned to close the dispenser body, to which it can be removably connected, and an inner channel, integral with the upper part of the cap, suited to house said tube or duct of the dispenser body.

The cylindrical wall of said inner channel of the cap comprises, on its lower edge, one or more teeth or projections suited to be inserted in corresponding through slots or holes made in said dispenser body.

A downward pressure exerted on said cap - correctly positioned - causes said teeth or projections to be completely or partially inserted in said slots, so that they rest on an element, hereinafter called valve-opening element, positioned between said dispenser body and the upper edge of the container, connected to said valve.

The pressure exerted on said valve-opening element causes said valve to open, which makes the fluid flow out through said tube or duct integral with the dispenser body.

The pad or filter partially inserted in said tube absorbs the fluid and conveys it to the top, getting completely soaked with said fluid.

In order to maximize the capacity of the pad to absorb and convey the coolant, according to the invention said pad or filter is made of cellulose or open-cell foam, preferably polyurethane S518.

The inner channel of the cap, which contains said tube completely, keeps the inserted pad or filter in the correct position, thus preventing it from being expelled due to the gas pressure.

Said cap or plug is made of a plastic material that has suitable mechanical, chemical and heat resistance, and is preferably transparent, so that the user, once having activated the cap and caused the fluid to flow out, can check from the outside if the pad is soaked and therefore ready to be used.

In fact, the outflow of fluid and the absorption of the same by the pad makes the condensate deposit on the inner surface of the cap near the pad itself, thus making it clear that the pad is soaked and ready to be used.

Said cap and/or said dispenser body and/or said container is/are provided with ribs and/or grooves for the correct alignment and positioning of the cap. In particular, according to the invention said cap can be aligned and arranged on said dispenser body in two different positions. In position "OFF", the cap applied to said dispenser body is rotated so that said teeth do not fit in the corresponding slots made in the dispenser body and therefore the fluid cannot be dispensed.

In position "ON", the cap is rotated for example by 180° with respect to position "OFF", so that said teeth are aligned with said slots, and so that by pressing the cap towards the container the user can insert said teeth in said slots and activate said valve-opening element, thus dispensing the fluid.

To guide the user and allow him/her to arrange the cap correctly in the closing or opening position, the two correct positions are indicated on the cap by means of marks, symbols or the like.

The characteristics of the new dispensing device will be highlighted in greater detail in the following description, with reference to the drawings attached as non-limiting examples.

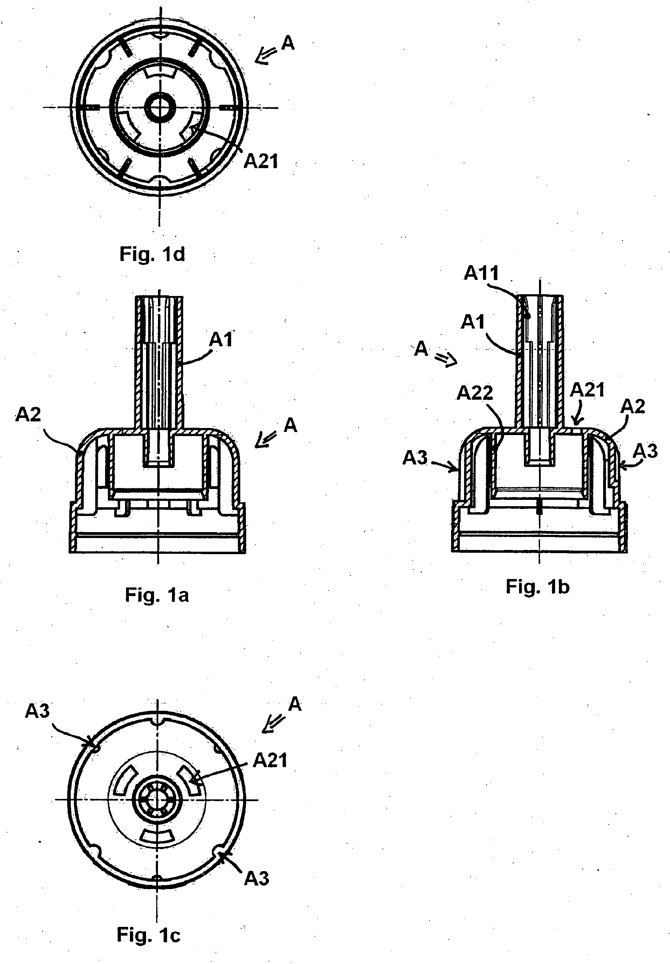

Figures 1a and 1b show two vertical sections of the dispenser body (A), while Figures 1c and 1d are a top view and a bottom view.

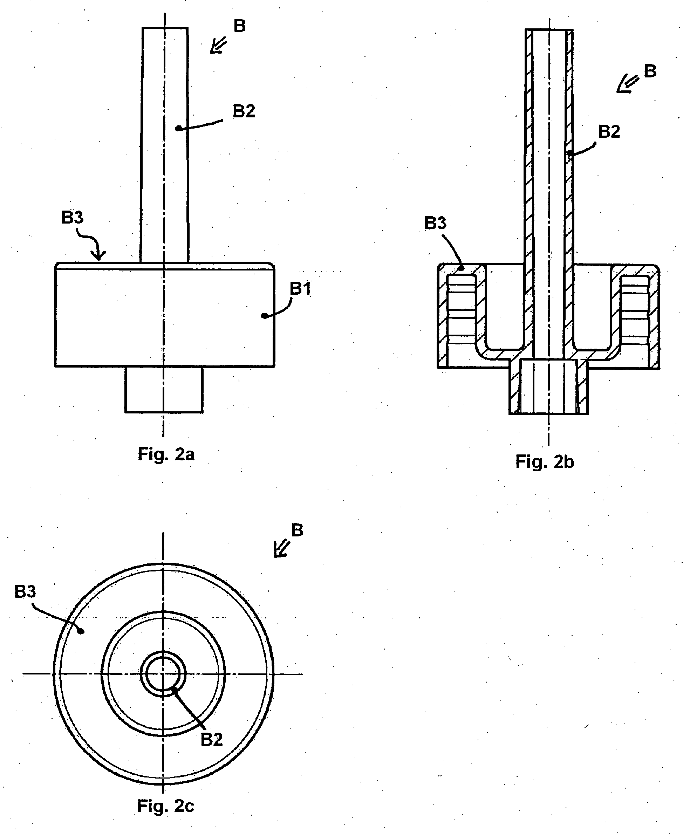

Figures 2a-2c show a side view, a cross section and a a bottom view of the valve-opening element (B) only.

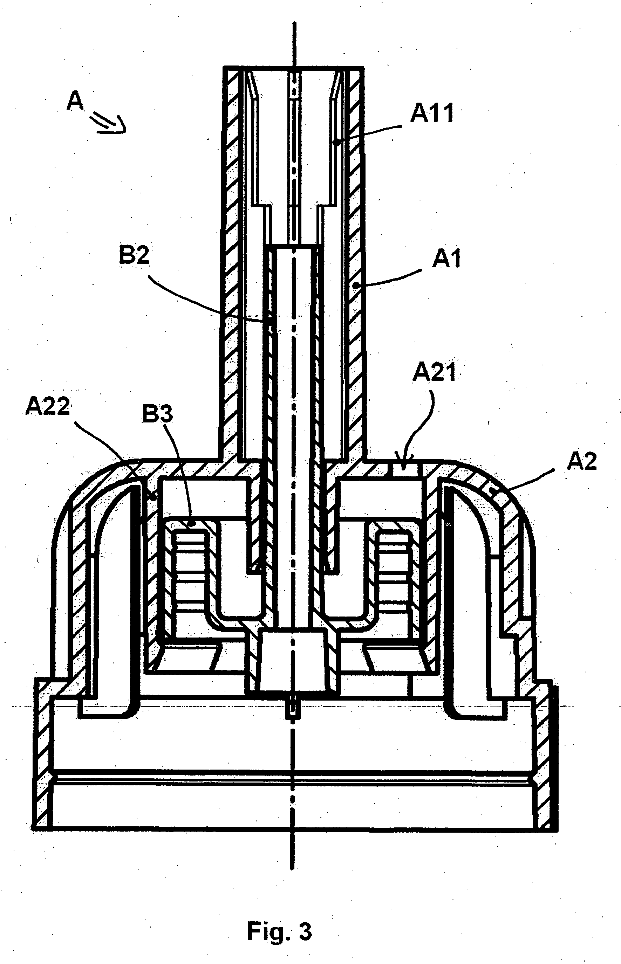

Figure 3 shows a section of the dispenser body (A) assembled with the valve-opening element (B).

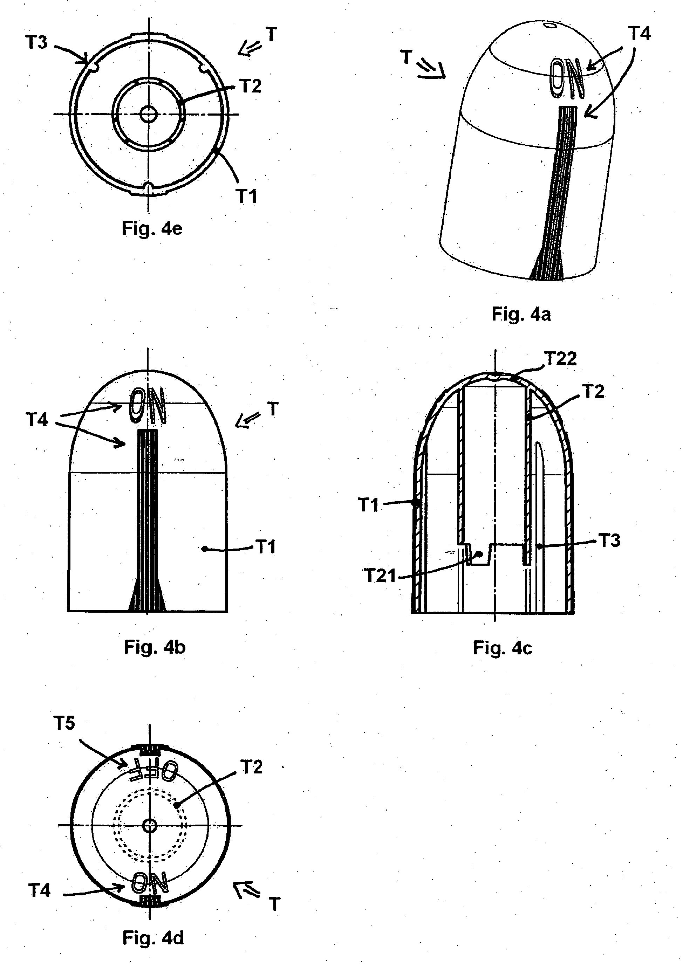

Figure 4a shows a 3D view of the activator cap (T) only, while Figures 4b-4e show a side view, a cross section, a top and a bottom view of the same.
Figures 5a-5c schematically represent the operation of the activator cap (T), which is in position "OFF" in Figure 5a and in position "ON" in Figures 5b and 5c.
Figures 6a and 6b show side views of the cap where it is possible to see the alignment of the reference marks present on the cap (T) and on the dispenser body (A), with the cap in position "OFF" and "ON", respectively.

The new dispensing device can be integrally applied to pressurized containers (C) equipped with a manual valve (C1), of the sprayer type, and comprises a dispenser body (A) suited to be fixed to said container (C). Said dispenser body (A) substantially comprises a convex or dome-shaped part (A2) suited to contain the upper end of the container and at least one duct or tube (A1) integral with and built in said cupola-shaped part (A2) for the outflow of the coolant contained in the container (C).

The inside of said tube (A1) is shaped in such a way as to comprise ribs (A11) suited to hold at least one cylindrical pad or filter (S) partially inserted in it.

In the preferred embodiment, in order to guarantee effective absorption and conveyance of the coolant, said cylindrical pad or filter (S) is made if polyurethane S518, with a diameter of approximately 7 mm and a length of 18 mm.

A valve-opening element (B) is interposed and fixed between said dispenser body (A) and said container (C), and comprises an annular portion (B1) inserted between partitions (A22) that are integral with the dispenser body (A) and at least one channel (B2) communicating with said valve (C1), at least partially inserted - coaxially - in said tube (A1) for application of the coolant.

Said valve-opening element (B) rests on said valve (C1) so that the downward travel of the valve-opening element (B) itself, for example caused by a downward force exerted on it, causes the opening of the valve (C1) and thus the outflow of the coolant that, through said channel (B2) provided inside said tube (A1), reaches said pad or filter (S) and is absorbed. The new dispensing device also includes at least one activator plug or cap (T) comprising a casing (T1) suited to be positioned to close the dispenser body (A), to which it can be removably connected, and an inner channel (T2), integral with the upper part of the cap (T), suited to house said tube (A1) of the dispenser body (A).

The cylindrical wall of said inner channel (T2) of the cap (T) comprises, on its lower edge, one or more teeth or projections (T21) suited to be inserted in corresponding through slots or holes (A21) made in said dome-shaped portion (A2) of said dispenser body (A), so that it can rest against a part (B3) of said valve-opening element (B), causing the valve (C1) to be opened and the fluid to be dispensed.

The outflow of fluid and the absorption of the same by the pad (S) makes the condensate deposit on the inner surface of the cap (T) in proximity of the top (T22) near the pad (S) itself, thus making it clear for the user that the pad is soaked.

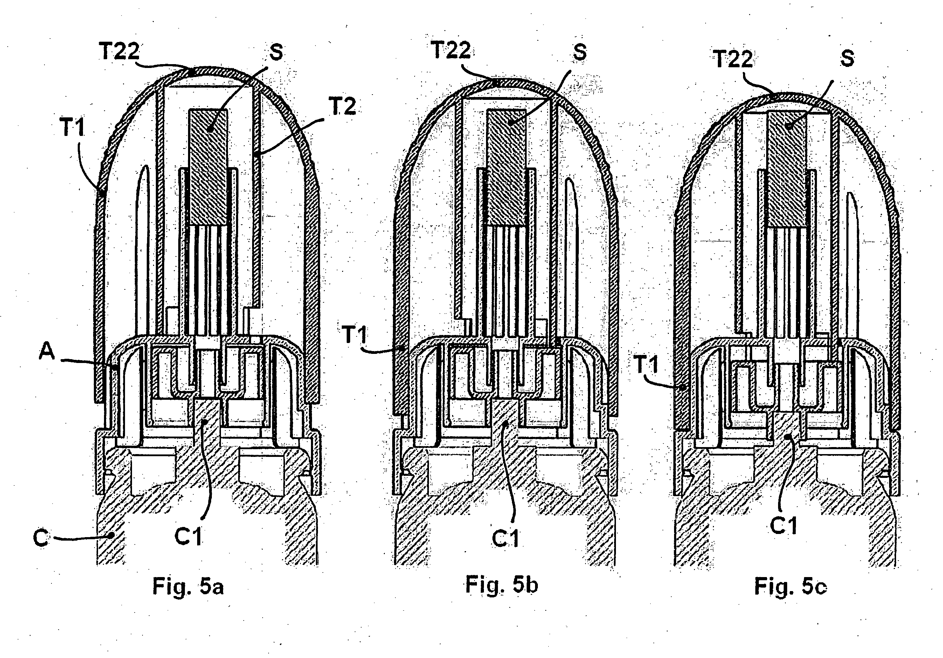

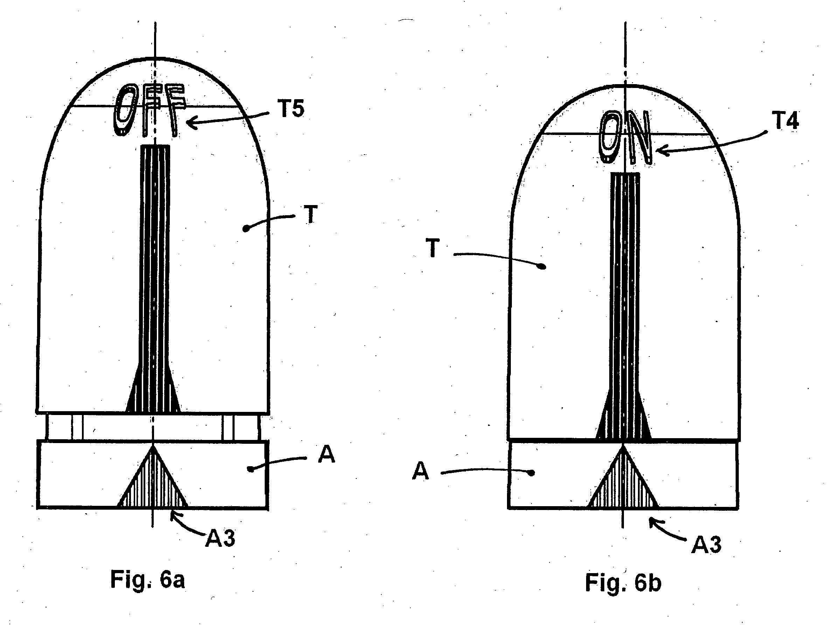

Furthermore, according to the invention, the inner surface of said casing (T1) of the cap (T) is provided with one or more ribs (T3), suited to be inserted in corresponding grooves present in said dispenser body (A), which serve as guides for the correct alignment and positioning of the cap (T) itself. In particular, according to the invention, said cap can be aligned and arranged on said dispenser body in two different positions. In position "OFF" (Figure 5a and 6a), the cap (T) inserted in said dispenser body is rotated so that said teeth (T21) are not aligned with said corresponding slots (A21) present in the dispenser body (A) and therefore they cannot be inserted in it and act on said valve-opening element (B).

This prevents the fluid from being dispensed.

In position "ON" (Figure 6b and 5b), the cap (T) is rotated by 180° with respect to position "OFF", so that said teeth (T21) are aligned with said slots (A21).

If a force directed towards the container (C) is exerted on said cap (T), as shown in Figure 5c, said teeth (T21) are inserted in the slots (A21) and, resting against said valve-opening element (B), cause the valve (C1) to be opened and the fluid to be dispensed.

To guide the user and allow him/her to correctly align the cap in position "OFF" or "ON", said cap (T) and said dispenser body (A) are provided with reference marks that show how to align the cap (T) correctly and define the two "OFF" and "ON" positions.

In particular, said cap (T) is provided with an arrow and/or a reference mark (T4) to indicate position "ON" and with an arrow and/or reference mark (T5) to indicate position "OFF", to be aligned with the reference mark (A3) provided on said dispenser body (A).

Therefore, with reference to the above description and the attached drawings, the following claims are expressed.

## Claims

1. Dispensing device for pressurized containers (C) of coolants for cryotherapy, comprising at least one manual opening/closing valve (C1), comprising:
- at least one dispenser body (A) suited to be permanently fixed to said container (C);
- at least one closing plug or cap (T) suited to be removably positioned on and fixed to said dispenser body (A);
**characterized in that** it comprises:
- at least one tube or duct (A1) for application of the coolant, at one end of which at least one applicator pad or filter (S) is removably inserted, said tube or duct (A1) being permanently built in and integral with said dispenser body (A);
- at least one valve-opening element (B) positioned between said dispenser body (A) and said container (C) and resting on said valve (C1);
- one or more teeth or projections (T21) on said cap (T) resting on said valve-opening element (B) to cause the valve (C1) to open;
- one or more through slots or holes (A21) on said dispenser body (A) for the insertion of one or more teeth or projections (T21) present on the lower edge of the internal channel (T2) of said cap (T), and wherein one or more of said teeth (T21), inserted through said slots (A21), rest on said valve-opening element (B) to cause the valve (C1) to be opened and the fluid to be dispensed;
- one or more ribs (T3) and/or grooves (A3) for the guided application with correct alignment of said cap (T) to said dispenser body (A), on said cap (T) and/or said dispenser body (A) and/or said container (C); and wherein said ribs (T3) and/or grooves (A3) allow said cap (T) to be applied to said dispenser body (A) and aligned in position "ON", so that said teeth (T21) are aligned with said slots (A21) and can thus be inserted in them by pressing the cap (T) towards the container (C) and in position "OFF", rotated by a given angle with respect to said position "ON", so that said teeth (T21) in position "OFF" are not aligned with said slots (A21) and thus cannot be inserted in them.

2. Dispensing device according to claim 1, **characterized in that** said cap (T) comprises an external casing (T1) that surrounds and encloses said dispenser body (A) and at least one internal channel (T2), integral with the upper part of the casing (T1), suitable for housing said tube (A1), and wherein the upper part (T22) of said cap (T) is in proximity of said pad or filter (S), partially inserted in said tube (A1), which is held in the correct position while the coolant is being dispensed.

3. Dispensing device according to claims 1, 2, **characterized in that** said cap (T) is completely or partially made of a transparent plastic material that makes it possible to see from the outside the condensate that forms when the coolant is dispensed.

4. Dispensing device according to the preceding claims, **characterized in that** said valve-opening element (B) comprises at least one channel (B2) communicating with the opening of said valve (C1), at least partially inserted in said tube (A1) and suited to convey said dispensed coolant and make it reach said pad (S) partially inserted in said tube (A1).

5. Dispensing device according to the preceding claims, **characterized in that** said pad or filter (S) is made of cellulose or open-cell foam or another material with high capacity to absorb and convey the coolant.

6. Dispensing device according to the preceding claims, **characterized in that** said cap (T) and/or said dispenser body (A) and/or said container (C) are provided with one or more cuts or symbols or reference marks (A3, T4, T5) for the correct alignment and arrangement of the cap (T) in position "OFF" or "ON".

## Patentansprüche

1. Ausgabevorrichtung für unter innerem Überdruck stehende Behälter (C) mit Kühlmitteln für die Kryotherapie, wenigstens ein manuelles Öffnungs- und Schließventil (C1) aufweisend, Folgendes umfassend:
- wenigstens einen Ausgabekörper (A), der geeignet ist, dauerhaft mit dem Behälter (C) verbunden zu werden;
- wenigstens einen Verschlussstopfen oder -deckel (T), dazu geeignet, abnehmbar am besagten Ausgabekörper (A) positioniert und befestigt zu werden,
**dadurch gekennzeichnet, dass** sie Folgendes umfasst:
- wenigstens eine Röhre oder Leitung (A1) zum Auftrag des Kühlmittels, an deren einem Ende wenigstens ein Applikatorkissen oder -filter (S) abnehmbar eingesetzt ist, wobei die besagte Röhre oder Leitung (A1) dauerhaft in den besagten Ausgabekörper (A) eingebaut ist und mit diesem eine Einheit bildet;
- wenigstens ein Ventilöffnungselement (B), das zwischen dem besagten Ausgabekörper (A) und dem besagten Behälter (C) positioniert ist und auf dem besagten Ventil (C1) aufliegt;
- einen oder mehrere Zähne oder Auskragungen (T21) an besagtem Stopfen (T), die auf dem besagten Ventilöffnungselement (B) aufliegen, um die Öffnung des Ventils (C1) zu bewirken;
- einen oder mehrere durchgehende Schlitze oder Löcher (A21) in dem besagten Ausgabekörper (A) zum Eingriff eines oder mehrerer Zähne oder Auskragungen (T21), die an der unteren Kante des Innenkanals (T2) des besagten Deckels (T) vorhanden sind, und wobei einer oder mehrere der besagten, durch die besagten Schlitze (A21) eingesteckten Zähne (T21) auf dem besagten Ventilöffnungselement (B) liegen, um die Öffnung des Ventils (C1) und die Ausgabe der Flüssigkeit zu bewirken;
- eine oder mehrere Rippen (T3) und/oder Rillen (A3), welche auf besagtem Deckel (T) und/oder auf besagtem Ausgabekörper (A) und/oder auf besagtem Behälter (C) vorhanden sind, zum gelenkten Auftrag mit korrekter Ausrichtung des besagten Deckels (T) auf den besagten Ausgabekörper (A), und wobei die genannten Rippen (T3) und/oder Rillen (A3) die Anbringung des besagten Deckels (T) an dem besagten Ausgabekörper (A) und seine Ausrichtung auf die Position "EIN" erlauben, so dass die besagten Zähne (T21) auf die besagten Schlitze (A21) ausgerichtet sind und somit in diese eingesteckt werden können, indem der Deckel (T) zum Behälter (C) gedrückt wird, und seine Ausrichtung auf die bezüglich der besagten Position "EIN" um einen gegebenen Winkel gedrehte Position "AUS", so dass die besagten Zähne (T21) in der Position "AUS" nicht auf die besagten Schlitze (A21) ausgerichtet sind und somit nicht in diese eingesteckt werden können.

2. Ausgabevorrichtung gemäß Patentanspruch 1, **dadurch gekennzeichnet, dass** der besagte Deckel (T) ein Außengehäuse (T1) umfasst, das den besagten Ausgabekörper (A) umgibt und umschließt, sowie wenigstens einen Innenkanal (T2), der eine Einheit mit dem oberen Teil des Gehäuses (T1) selbst bildet und geeignet ist, die besagte Röhre (A1) aufzunehmen, und wobei der obere Teil (T22) des besagten Deckels (T) sich in der Nähe des besagten Kissens oder Filters (S) befindet, der teilweise in die besagte Röhre (A1) eingefügt ist, welche in korrekter Position gehalten wird, während das Kühlmittel ausgegeben wird.

3. Ausgabevorrichtung gemäß Patentansprüchen 1, 2, **dadurch gekennzeichnet, dass** der besagte Deckel (T) ganz oder teilweise aus einem transparenten Kunststoff hergestellt ist, der es ermöglicht, das sich beim Ausgeben des Kühlmittels bildende Kondensat von außen zu sehen.

4. Ausgabevorrichtung gemäß vorstehenden Patentansprüchen, **dadurch gekennzeichnet, dass** das besagte Ventilöffnungselement (B) wenigstens einen Kanal (B2) umfasst, der mit der Öffnung des besagten Ventils (C1) kommuniziert, wenigstens teilweise in die besagte Röhre (A1) eingefügt und geeignet ist, das besagte, ausgegebene Kühlmittel zu leiten und das besagte Kissen (S) erreichen zu lassen, das teilweise in die besagte Röhre (A1) eingesetzt ist.

5. Ausgabevorrichtung gemäß vorstehenden Patentansprüchen, **dadurch gekennzeichnet, dass** das besagte Kissen bzw. der besagte Filter (S) aus Zellulose oder offenporigem Schaumstoff oder einem anderen Material hergestellt ist, das eine hohe Kapazität besitzt, das Kühlmittel aufzunehmen und zu leiten.

6. Ausgabevorrichtung gemäß vorstehenden Patentansprüchen, **dadurch gekennzeichnet, dass** der besagte Deckel (T) und/oder der besagte Ausgabekörper (A) und/oder der besagte Behälter (C) eine oder mehrere Einschnitte oder Symbole oder Bezugskerben (A3, T4, T5) für die korrekte Ausrichtung und Anordnung des Deckels (T) in Position "AUS" oder "EIN" aufweisen.

## Revendications

1. Dispositif distributeur pour récipients pressurisés (C) de fluides réfrigérants pour la cryothérapie, comprenant au moins une soupape (C1) d'ouverture/fermeture manuelle, comprenant :
- au moins un corps distributeur (A) apte à être fixé de manière permanente audit récipient (C) ;
- au moins un bouchon (T) ou un capuchon de fermeture apte à être positionné et fixé de manière amovible sur ledit corps distributeur (A) ;
**caractérisé en ce qu'**il comprend :
- au moins un tube ou conduit (A1) pour l'application du fluide réfrigérant, sur une extrémité duquel au moins un tampon ou filtre applicateur (S) est inséré de manière amovible, ledit tube ou conduit (A1) étant intégré et solidaire de manière permanente dudit corps distributeur (A) ;
- au moins un élément d'ouverture de soupape (B) positionné entre ledit corps distributeur (A) et ledit récipient (C) et appuyé sur ladite soupape (C1) ;
- une ou plusieurs dents ou saillies (T21) sur ledit bouchon (T), appuyées sur ledit élément d'ouverture de soupape (B) afin de réaliser l'ouverture de la soupape (C1);
- une ou plusieurs fentes ou trous passants (A21) présents sur ledit corps distributeur (A), pour l'introduction d'un ou plusieurs desdites dents ou saillies (T21) réalisées sur le bord inférieur du conduit intérieur (T2) dudit bouchon (T), et où un ou plusieurs desdites dents (T21), insérées à travers lesdites fentes (A21), s'appuient sur ledit élément d'ouverture de soupape (B) afin de réaliser l'ouverture de la soupape (C1) et la distribution du fluide réfrigérant ;
- une ou plusieurs nervures (T3) et/ou rainures (A3) pour l'application guidée avec un alignement correct dudit bouchon (T) sur ledit corps distributeur (A), présentes sur ledit bouchon (T) et/ou sur ledit corps distributeur (A) et/ou sur ledit récipient (C) ; et où lesdites nervures (T3) et/ou rainures (A3) permettent l'application et l'alignement dudit bouchon (T) sur ledit corps distributeur (A) en position d'ouverture, de façon à ce que lesdites dents (T21) soient alignées avec lesdites fentes (A21) et que puissent donc être insérées dans celles-ci par pression sur le bouchon (T) vers le récipient (C), et en position de fermeture, tournée d'un angle déterminé par rapport à ladite position d'ouverture, de façon à ce que lesdites dents (T21) en position de fermeture ne soient pas alignées avec lesdites fentes (A21) et donc ne puissent pas être insérées dans celles-ci.

2. Dispositif distributeur, selon la revendication 1, **caractérisé en ce que** ledit bouchon (T) comprend une enveloppe extérieure (T1) d'enroulement et fermeture dudit corps distributeur (A) et au moins un conduit intérieur (T2), solidaire de ladite partie supérieure de l'enveloppe (T1), adapté au logement dudit tube (A1), et où la partie supérieure (T22) dudit bouchon (T) est proche dudit tampon ou filtre (S), partiellement inséré dans ledit tube (A1), qui est retenu dans la position correcte durant la distribution du fluide réfrigérant.

3. Dispositif distributeur, selon les revendications 1, 2, **caractérisé en ce que** ledit bouchon (T) est réalisé complètement ou en partie en matériel plastique transparent qui le rend visible de l'extérieur du condensat qui se forme au moment de la distribution du fluide réfrigérant.

4. Dispositif distributeur, selon les revendications précédentes, **caractérisé en ce que** ledit élément d'ouverture de soupape (B) comprend au moins un conduit (B2) communiquant avec l'ouverture de ladite soupape (C1), au moins partiellement inséré dans ledit tube (A1) et apte à transporter ledit fluide réfrigérant distribué jusqu'audit tampon (S) partiellement inséré dans ledit tube (A1).

5. Dispositif distributeur, selon les revendications précédentes, **caractérisé en ce que** ledit tampon ou filtre (S) est réalisé en cellulose ou mousse à cellules ouvertes ou autre matériel à haute capacité d'absorption et de transport du fluide réfrigérant.

6. Dispositif distributeur, selon les revendications précédentes, **caractérisé en ce que** ledit bouchon (T) et/ou ledit corps distributeur (A) et/ou ledit récipient (C) présentent une ou plusieurs incisions ou symboles ou autre type d'indications (A3, T4, T5) pour l'alignement et positionnement corrects du bouchon (T) en position de fermeture ou d'ouverture.
